# EUROPEAN PATENT SPECIFICATION

(11) **EP 3 155 955 B1**
(45) Date of publication and mention of the grant of the patent: **27.03.2019**
(21) Application number: 16193969.9
(22) Date of filing: 14.10.2016
(51) Int. Cl.: A61B 1/00

(54) **ENDOSCOPE CAP WITH SEPARABLE ARMS**
ENDOSKOPKAPPE MIT ABNEHMBAREN ARMEN
CAPUCHON D'ENDOSCOPE AVEC BRAS SÉPARABLES

(30) Priority: 14.10.2015 US 201562241423 P
(43) Date of publication of application: 19.04.2017
(73) Proprietor: Cook Medical Technologies LLC, Bloomington, IN 47404 (US)
(72) Inventor: PETROSKEY, David, Lorain, OH Ohio 44053 (US)
(74) Representative: Garratt, Peter Douglas

(56) References cited:
- EP-A1- 2 596 741
- WO-A1-2015/077684
- JP-A- 2012 040 108
- US-A1- 2002 169 362
- US-A1- 2007 197 871
- US-A1- 2013 102 843
- US-A1- 2014 358 089

## Description

### BACKGROUND

Endoscopic devices and procedures may be used to diagnose, monitor and treat various conditions by close examination of the internal organs. By way of background, a conventional endoscope generally is an instrument having a device for visualizing the interior of an internal region of a body and a lumen for inserting one or more treatment devices therethrough. A wide range of applications have been developed for the general field of endoscopes including by way of example the following: arthroscope, angioscope, bronchoscope, choledochoscope, colonoscope, cytoscope, duodenoscope, enteroscope, esophagogastro-duodenoscope (gastroscope), laparoscope, laryngoscope, nasopharyngo-neproscope, sigmoidoscope, thoracoscope, and utererscope (individually and collectively, "endoscope").

An endoscope may be useful to treat one of the several disorders of the gastrointestinal tract, e.g., gastrointestinal inflammation, gastrointestinal cancer, gastrointestinal infection, gastrointestinal motility dysfunction, or lesions, wounds or contusions of tissue of a portion of the gastrointestinal tract that can cause gastrointestinal lesions. There is a wide variety of medical procedures that require removal or dissection of the mucosal or submucosal layers of gastrointestinal tract wall to treat these disorders.

To treat motility disorders from within the esophagus, a procedure called per-oral endoscopic myotomy ("POEM") may be used. In this procedure, a tunnel is generally formed beneath the mucosal and submucosal layers such that a delivery device (e.g., an endoscope with a working channel) may access the underlying layers of muscle tissue. The top muscle layer may then be cut in the axial direction, which may weaken tightness in the esophagus to treat the motility disorder.

Currently, a high level of skill and attention is required access the muscle layer. The target area where it is desirable to perform the cut is near vital arteries and is generally at a location in a position of the body that is difficult to reach, and therefore it is difficult for a medical professional to precisely operate at the target location. Further, because several layers of tissue, such as mucosal and submucosal layers, may cover the target muscle area, viewing devices may be blocked at the target site, thereby severely limiting the visual feedback.

Accordingly, it would be desirable to provide an improved device located at the end of an endoscope which may assist in tunneling through tissue layers, may facilitate uncovering of a target area for performance of a medical procedure, and may improve the visual feedback provided to a medical professional.

Reference is directed to WO 2015/077684 which discloses colposcopes having light emitters and image capture devices and associated methods. According to an aspect, a colposcope includes an elongate body having a distal end, a proximate end, and an axis extending between the distal end and the proximate end. A cavity expander is formed at the distal end by two members together with an actuating mechanism that can move the members from a closed position, in which they together form a tubular shape, to an open position. The actuating mechanism has an actuating ring and two wires connected respectively with the two members.

Reference is further directed to US 2014/0358089 which discloses devices for facilitating access to the pancreatiocobiliary system and in particular, to devices used to apply suction to the papilla, e.g., duodenal tissue surrounding the papilla, to facilitate cannulation to reach the bile duct and/or pancreatic duct. Devices may include an endoscope cap configured to apply suction to a tissue surface. The cap may include doors which can pivot or swing open to engage a tissue surface, with a pull wire or push rod extending through the cap and the endoscope.

### BRIEF SUMMARY

The present invention provides a cap as defined by claim 1 for attachment to a distal end of an endoscope. The cap comprises a proximal portion comprising a tubular body with a lumen extending therethrough, wherein the proximal end is configured for securement to the distal end of the endoscope. The cap further comprises a distal portion having a first arm extending to a first distal end, the first arm being movable such that a first end of the first arm is moveable laterally with respect to a longitudinal axis defined by the tubular body.

The cap has activator in contact with a first surface of the first arm, the activator being moveable to effect the movement of the first distal end of the first arm The cap comprises a spring circumnavigating the distal portion of the cap and configured to provide an inward lateral force on the first arm towards the longitudinal axis defined by the tubular body.

The distal portion of the cap may further comprise a second arm, the second arm being movable such that a second distal end of the second arm is moveable laterally with respect to the longitudinal axis defined by the tubular body. The cap may have an activator disposed at least partially between the first arm and the second arm. A portion of the activator is configured to slideably contact the first surface of the first arm.

### BRIEF DESCRIPTION OF THE DRAWINGS

FIG. 1 is a front view of a cap for attachment to a distal end of an endoscope in an open configuration in accordance with an embodiment of the present invention.
FIG. 2 is a front cutout view of a cap for attachment to a distal end of an endoscope with a first arm in accordance with an embodiment of the present invention.
FIG. 3 is a front view of a cap attached to the distal end of an endoscope and in an open configuration in an esophagus of a patient in accordance with an embodiment of the present invention.
FIG. 4 is a side, rotated view of a cap for attachment to a distal end of an endoscope in accordance with an embodiment of the present invention.
FIG. 5 is a front view of a cap for attachment to a distal end of an endoscope in a closed configuration in accordance with an embodiment of the present invention.
FIG. 6 is a front view of a cap for attachment to a distal end of an endoscope in an open configuration in accordance with an embodiment of the present invention.

### DETAILED DESCRIPTION OF THE DRAWINGS

The invention is described with reference to the drawings in which like elements are referred to by like numerals. The relationship and functioning of the various elements of this invention are better understood by the following detailed description. However, the embodiments of this invention are not limited to the embodiments illustrated in the drawings. It should be understood that the drawings are not to scale, and in certain instances details have been omitted which are not necessary for an understanding of the present invention, such as conventional fabrication and assembly.

As used in the specification, the terms proximal and distal should be understood as being in the terms of a physician delivering cap to a patient. Hence the term "distal" means the portion of the cap that is farthest from the physician and the term "proximal" means the portion of the cap that is nearest to the physician.

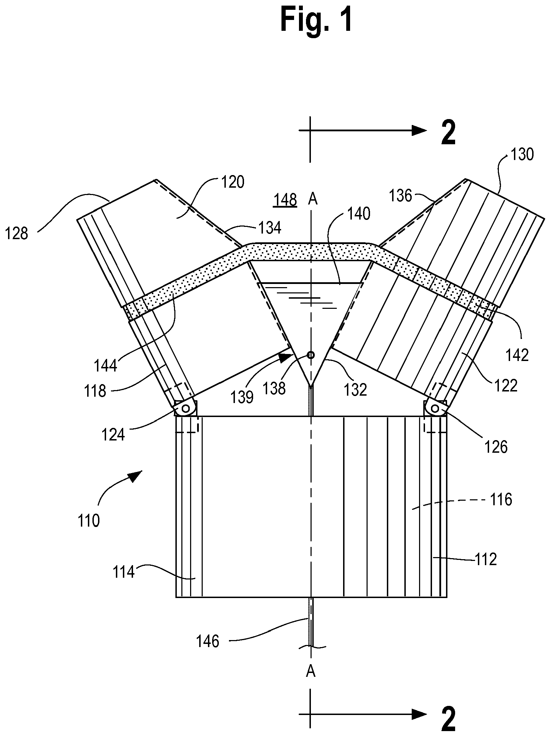

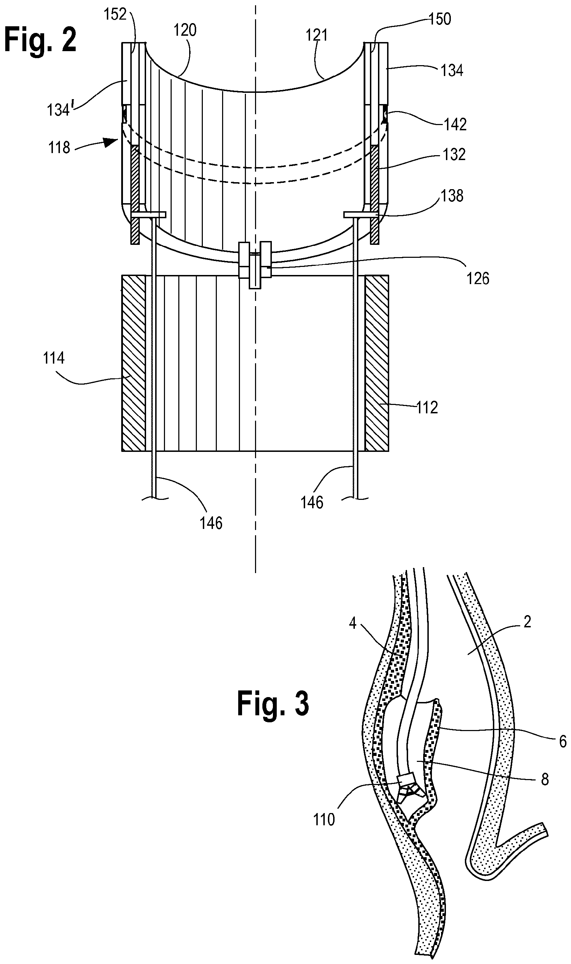

FIG. 1 illustrates one embodiment of a cap 110. As shown in FIG. 1, the cap 110 may include a tubular body 114 at a proximal portion 112 of the cap 110, the tubular body 114 having a lumen 116 formed therein. The proximal portion 112 may be secured to a medical device, such as an endoscope 102 (see FIG. 3), and may be configured such that the tubular body 114 fits over the distal end of the endoscope. In some embodiments, additional suitable devices may be included and configured to facilitate this described securement, such as an elastomeric portion as described by U.S. Patent Application Publication 2014/0100570 to McLawhorn, which is incorporated by reference herein in its entirety. Further, the cap 110 may be a multipart cap being universally connectable to many different diameter endoscopes, as described by U.S. Patent Application Publication 2013/0046138, also to McLawhorn, which is incorporated by reference herein in its entirety.

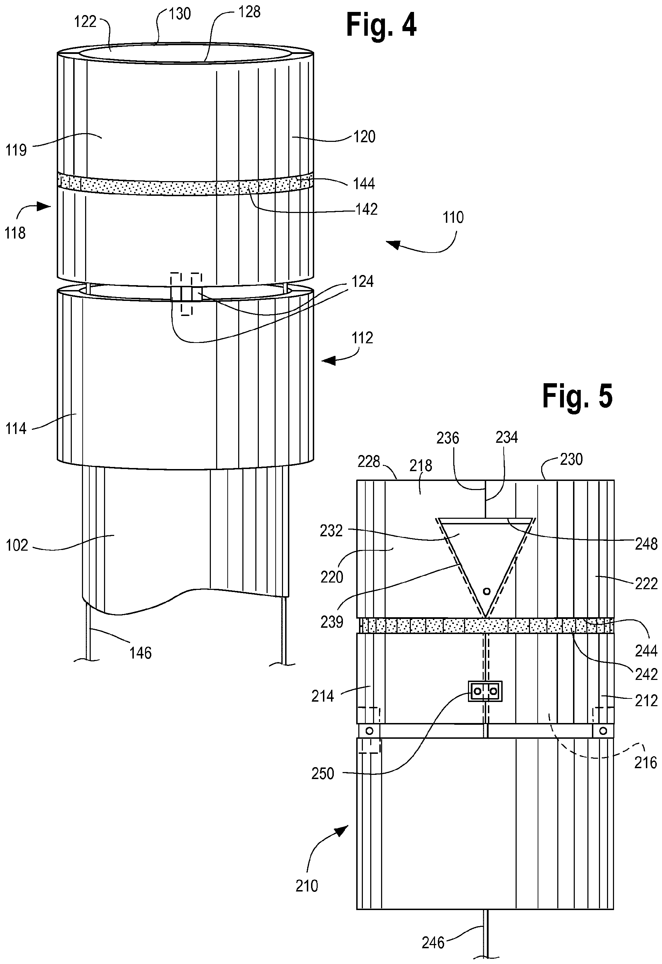

Referring to the embodiment of FIG. 1, the cap 110 comprises a distal portion 118 having a first arm 120 and a second arm 122. The first arm 120 and the second arm 122 may be connected to the proximal portion 112 through a first hinge 124 and second hinge 126, respectively. The distal portion 118 (and potentially at least a portion of the proximal portion 112) may extend distally from the endoscope 102 (see FIG. 4). In other embodiments, only one arm or more than two arms may be provided. The first arm 120 may extend to a first distal end 128, and the second arm 122 may have a second distal end 130. The first arm 120 may be moveable (e.g., may be pivotable at first hinge 124) such that the first distal end 128 is movable laterally (i.e., substantially radially towards and away an axis) with respect to a longitudinal axis A defined by the tubular body 114. Similarly, the second arm 122 may be moveable (e.g., may be pivotable at second hinge 126) such that the second distal end 130 is moveable laterally with respect to the longitudinal axis A of the tubular body 114. In other words, the arms 120 and 122 may comprise an open state (depicted in FIG. 1) and a closed state (depicted in FIGS. 4 and 5), wherein in the open state, the distal ends 128 and 130 are positioned farther away from the longitudinal axis A defined by the tubular body 114 than they are in the closed state. One or more selectable intermediate states may also be possible. In some embodiments, the arms 120, 122 may be cooperatively laterally movable to the open state. Further, the first arm 120 or the second arm 122 may be positioned a different distance away from the longitudinal axis A than the other of the first arm 120 and the second arm 122 in the open state (or any other state). In the closed state depicted by FIG. 4, the cap 110 may be substantially tubular with lumen 116 extending through the proximal portion 112 and the distal portion 118. As shown, the arms 120, 122 may be sized and shaped in a curved configuration so that surfaces 134, 136 of the arms 120, 122 meet in the closed state and form a second tubular body 119. In some embodiments, the distal portion 118 in the closed state may have an outer diameter substantially equal to an outer diameter of the proximal portion 112. In other embodiments, the outer diameter of the distal portion 118 may be smaller than or greater than the outer diameter of the proximal portion 112.

In some embodiments, certain portions of the cap 110, including, for example, the tubular body 114 and the arms 120 and 122, may be made primarily of a substantially transparent or translucent polymer such as polytetrafluorothylene (PTFE). Additional possible materials include, but are not limited to the following, polyethylene ether ketone (PEEK), fluorinated ethylene propylene (FEP), perfluoroalkoxy polymer resin (PFA), polyamide, polyurethane, high density or low density polyethylene, and nylon. In some embodiments, the cap may be formed from a lubricious material such as PTFE and the like for easy slidability within the patient's lumen for delivery to the treatment site. The cap or a portion thereof may also be coated or impregnated with other compounds and materials to achieve the desired properties. Exemplary coatings or additives include, but are not limited to, parylene, glass fillers, silicone hydrogel polymers and hydrophilic coatings.

As shown in FIG. 1, the cap 110 may include an activator 132 with at least one contact portion 139. The activator 132 may be shaped as a wedge, as shown. The contact portion 139 may be, for example, a contact surface. The activator 132 may be operable to separate the arms 120 and 122 and/or to control the orientation of the arms 120 and 122, as shown in FIG. 1. When the arms 120 and 122 are separated, a space 148 may be provided. In some embodiments, at least a portion of this space 148 (e.g., between surfaces 134 and 136) may be covered by, for example, a cloth or another material which is preferably (though not necessarily) transparent. In other embodiments (for example, where only one arm is provided/movable), the activator 132 may separate a single arm from, for example, a stationary portion of the distal portion 118. The activator 132 may be moveable in the distal and proximal directions with respect to at least one of the arms 120 and 122, and may include a contact portion 139 is slideable relative to a surface of an arm, such as a first surface 134 of the first arm 120 and/or a second surface 136 of the second arm 122. In the depicted embodiment, when the activator 132 moves proximally, the contact portion 139 provides a separating force against the surfaces 134 and 136, thereby moving the distal ends 128 and 130 laterally away from the longitudinal axis A. The activator 132 may be generally triangular shaped, as depicted in FIG. 1, and may comprise a narrow portion 138 facing proximally and a wide portion 140 facing distally. The shape and size of the activator 132 may determine the range of the movement of the arms 120 and 122.

More than one activator may be provided. For example, in some embodiments, an activator may be provided at both locations where the first arm 120 and second arm 122 split (e.g., the front and back of the cap 110 from the perspective of FIG. 1). In some embodiments, a single activator may pass across the lumen 116 to act at both locations where the first arm 120 and second arm 122 split, though in other embodiments, the activator 132 is sized to avoid substantially extending from surfaces 134 and 136, which may prevent substantially interfering with working space of the device. In the depicted embodiment of FIG. 1, the activator 132 is symmetric so that the first arm 120 and the second arm 122 move equal distances away from and toward the longitudinal axis A when the activator 132 is move proximally and distally, respectively. In other embodiments, the activator 132 may be asymmetric so that the first arm 120 and the second arm 122 move different distances away from and toward the longitudinal axis A when the activator 132 is move proximally and distally, respectively. Further, the cap may include markings to provide an indication of how far the arms 120, 122 are opened. In some instances, there may be different optimal distances for movement from or toward the longitudinal axis A, and therefore providing markings or other forms of indicators may be advantageous to help an operator know the location of the arms 120, 122 and provide necessary adjustments to achieve optimal positioning. These markings or other types of indicators may be provided on the cap 110 (such as on the lumen 116 or the cable 146) or may be provided on an attached device (such as the endoscope). The markings may be associated with markings on the cable 146.

In some embodiments, and referring to FIG. 1 and FIG. 2, at least a portion of the activator (e.g., a contact portion 139, shown in FIG. 1) may be configured to fit within a groove 150 located at the surface 134 of the first arm 120 (shown in FIG. 2). The second arm 122 may comprise a similar groove. In some embodiments, a portion of the activator 132, such as contact portion 139, is slideable within the groove 150. The groove 150 preferably retains the activator 132 such that the activator does not separate from the arm 120, and may provide securement of the activator 132 to other elements of the cap 110. The contact between the contact portion 139 of the activator 132 and the groove 150 may be lubricated in some embodiments. In embodiments comprising two activators, a second groove 152 may be included on a depicted surface 134' of the arm 120 opposite of surface 134. Similarly, the second arm 122 may comprise two grooves on two surfaces or edges. Alternatively, or in addition, a groove could be located on an inner surface 121 of the first arm 120. In other embodiments, the activator 132 may be connected to one or more arms without the use of a groove and/or by utilizing another suitable connection device or method. Further, there may be a spring housed in the groove 150 to provide a bias on the contact portion 139 of the activator 132.

As depicted in FIG. 1, the distal portion 118 may comprise a spring 142 surrounding an outer perimeter of the distal portion 118. The spring 142 may be configured to provide a constricting force on the arms 120 and 122, which may bias the arms 120 and 122 toward a closed position (shown in FIG. 4). The spring 142 may be seated in a groove 144 located on the outer surfaces of arms 120 and 122, as shown. In some embodiments, the spring 142 may be covered to prevent snagging or potential damage to tissue adjacent to the spring 142. Alternatively, the spring 142 may be contained within a cavity formed by the arms 120 and 122, or may be located adjacent to the inner surface of the arms 120 and 122.

As shown in FIG. 1, the activator 132 may be attached to a cable 146, which may be controllable by a medical professional operating the cap 110. In some embodiments, the cable 146 may extend to the proximal end of the endoscope 102 (see FIG. 4). The cable 146 may be configured to pull the activator 132 proximally or otherwise provide a force on the activator 132 such that the activator 132 moves proximally to open the arms 120 and 122. In some embodiments, the cable 146 may be sufficiently ridged as to be capable of also providing a force in the distal direction on the activator 132, which may provide an operator with the ability to directly control distal movement of the activator 132 to thereby close the arms 120 and 122. The cable 146 may be located at least partially within the lumen 116, and may extend proximally of the tubular body 114 through the lumen of the endoscope. Other embodiments may provide a cable located on the outside of the tubular body 114.

FIG. 3 depicts the cap 110 in the open configuration within a submucosal tunnel 8 in an esophagus 2. The submucosal tunnel 8 may be formed between the mucosal and submucosal layers 6 and underlying muscle tissue 4. The arms (see FIG. 1) of cap 110 are shown as providing a working and visual space 148 distally of the endoscope 102, which may, as described herein, increase the ability of an operator to visualize within the space 148 and adjacent tissue, as well as provide additional space for the operation of a medical procedure (such as a POEM procedure) at space 148.

FIG. 4 depicts an embodiment of the cap 110 in a closed configuration. The cap 110 comprises the proximal portion 112 with the tubular body 114 connected to an endoscope 102. The distal portion 118 comprises the first arm 120 and the second arm 122 depicted in a closed configuration. In the closed configuration, the first distal end 128 of the first arm 120 and the second distal end 130 of the second arm 122 may contact or otherwise be adjacent to one another. Further, in the closed configuration, the distal portion 118 may form a substantially tubular body 119 with approximately the same diameter as the tubular body 114 of the proximal portion 112. Spring 142, which is seated in a groove 144, circumnavigates the distal portion 118, thereby biasing the arms 120 and 122 to remain in the closed configuration. The arms 120 and 122 may be, as described above, attached to the proximal portion 112 at hinges (e.g., the first arm 120 is attached at hinges 124). In FIG. 4, the activator 132 is hidden, though it should be noted that the cap 110 may be made of a transparent or translucent material, as described above.

Referring to FIG. 5, in some embodiments, the cap 210 may comprise a distal portion 218 with a first arm 220 and a second arm 222 forming a cavity 248, which is shaped to surround an activator 232. The cavity 248 may house the activator 232 when, for example, the first arm 220 and the second arm 222 are in a closed orientation (as depicted). This embodiment may be advantageous, as the activator 232 does not need to be positioned distally of the distal portion 218 when the arms 220 and 222 are in the described closed orientation. The cavity 248 may be located at any position along depicted separable surfaces 234 and/or 236, such as approximately in the longitudinal middle of the distal portion 218 (as depicted), though providing the cavity 248 adjacent to or near a first distal end 228 of the first arm 220 and/or a second distal end 230 of the second arm 222 may maximize the range of movement of the first and second arms 220 and 222.

As previously described, a spring 242, which rests in a groove 244, may provide a tendency for the arms 220 and 222 to close when the activator 232 is not forcing them towards an open orientation. In some embodiments, the activator 232 is sufficiently movable (e.g., a contact surface 239 is sufficiently slidable along surfaces 234 and 234) such that, when force is released from a cable 246, the spring 242 may provide a sufficient constricting force, which can be mechanically translated into a distal force contact surface 239 from the surfaces 234 and 236, to move the activator 232 upwards into cavity 248, thereby closing the arms 220 and 222. In other embodiments, the activator 232 may be forced upward a rigid cable 246 (as described above) or by another suitable device or method configured to move the activator 232 such that a closed and/or intermediate configuration can be achieved.

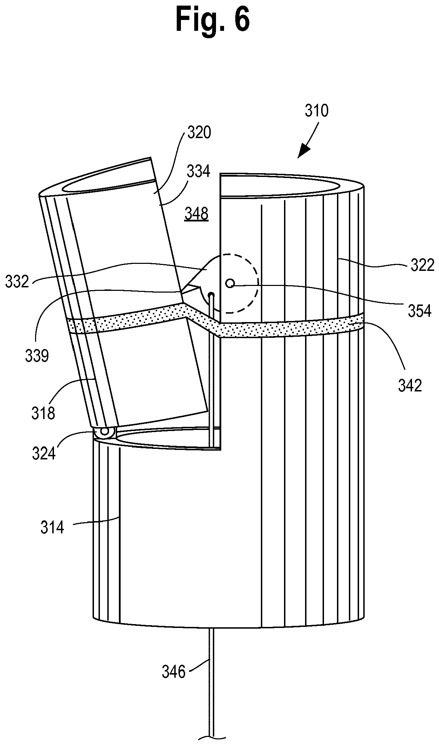

While the activator can operate as a wedge, as described above, it can alternatively and/or additionally be any other suitable device. As an illustration, in an alternative embodiment depicted in FIG. 6, a cap 310, shown in an open configuration, may comprise a rotatable activator 332. The activator 332 could be attached anywhere on the inside or outside of the cap 310. The rotatable activator 332 may be configured to separate a first arm 320 from a second arm 322 located on a distal portion 318 of the cap 310. As shown, the second arm 322 is integral to the proximal portion 314, and the first arm 320 is connected to the proximal portion 314 by way of a hinge 324. Alternatively, the second arm 322 could also be secured to the proximal portion 314 in a movable manner. The activator 332 is rotatably connected to the second arm 320 at a pivot point 354. A contact portion 339 of the activator 332 may be configured to contact a surface 334 of the first arm 320 to thereby effect the surface of the first arm 320.

Movement (e.g., rotation) of the activator 332, which may be influenced by a cable 346 controlled by an operator, can thereby influence the movement of the first arm 320 between a closed and the depicted open configuration. As depicted, the first arm 320 is in an open configuration and provides a space 348. The contact portion 339 may be enlarged, or may extend radially, such that it can contact the surface 334 (and in some embodiments, the contact portion 339 may extend radially and remain between the first arm 320 and the second arm 322 in intermediate states and the closed state). In some embodiments, the contact portion 339 may be slideably retained within an elongated groove or cavity of the surface 334 (not shown), and may therefore be capable of effecting the motion of the first arm 320 in two directions. As described above, a spring 342 circumnavigates the distal portion 318 and is configured to bias the distal portion 318 into the closed configuration. In addition, a rotation spring (e.g., a torsion spring, not shown) may be included and may create a bias for the activator to rotate into a position corresponding with the closed configuration.

The embodiments described herein have several advantageous characteristics. The separable arms described herein may separate such that they provide a force on two layers of adjacent tissue to facilitate separating those two layers during, for example, a POEM procedure, which may thereby facilitate tunneling under a tissue layer (e.g., the submucosa). Further, the separable arms may open and spread laterally, as described herein, to push tissue laterally and give exposure to underlying muscle, thereby allowing access by other instruments associated with the endoscope configured, for example, to perform a POEM procedure. This may additionally improve the view of a target area when using a device for visualizing the interior of an internal region of a body, particularly in embodiments where the arms and/or the other portions of the cap are made of a transparent or translucent material. The device further may be operated by a single medical professional, which may ease the process and reduce the need for assistance by a second user.

The figures and disclosure are intended to be illustrative and not exhaustive. This description will suggest many variations and alternatives to one of ordinary skill in the art. All such variations and alternatives are intended to be encompassed within the scope of the attached claims. Those familiar with the art may recognize other equivalents to the specific embodiments described herein which equivalents are also intended to be encompassed by the attached claims.

## Claims

1. A cap (110) for attachment to a distal end of an endoscope, the cap comprising:
a proximal portion (122) comprising a tubular body (114) with a lumen (116) extending therethrough, wherein the proximal portion is configured for securement to the distal end of the endoscope;
a distal portion (118) having a first arm (120) extending to a first distal end, the first arm being movable such that a first distal end of the first arm is moveable laterally with respect to a longitudinal axis defined by the tubular body; and
an activator (132) in contact with the first arm, wherein the activator is moveable to effect the movement of the first distal end of the first arm;
**characterized in that**:
a contact portion (139) of the activator is configured to slidably contact a first surface of the first arm; and
the cap further comprises a spring (142) circumnavigating the distal portion of the cap and configured to provide an inward lateral force on the first arm towards the longitudinal axis defined by the tubular body.

2. The cap of claim 1, wherein the distal portion of the cap further comprises a second arm (122), the second arm being movable in response to movement of the activator such that a second distal end of the second arm is moveable laterally with respect to the longitudinal axis defined by the tubular body.

3. The cap of claim 2, wherein the activator is disposed at least partially between the first arm and the second arm, wherein the contact portion of the activator is movable in the proximal direction to laterally spread the first distal end of the first arm from the second distal end of the second arm.

4. The cap of any of claims 1 to 3, wherein the activator is connected to a cable (146), the cable being configured to provide a force on the activator in a proximal direction.

5. The cap of claim 4, wherein the cable extends to a proximal end of an endoscope.

6. The cap of any of claims 1 to 5, wherein the activator includes the shape of a wedge.

7. The cap of any of claims 1 to 6, wherein the activator (132) is coupled to the second arm, and wherein the activator is rotatable.

8. The cap of claim 7, wherein the contact portion (139) of the activator (132) includes a contact surface configured to contact and provide a force to the first arm to laterally separate the first arm from the second arm.

9. The cap of any of claims 1 to 8, wherein the first arm comprises a hinge (124), the hinge being secured to the proximal portion of the cap and allowing lateral movement of the first distal end of the first arm.

10. The cap of claim 1, wherein the spring is received by a groove (150) circumnavigating the distal portion of the cap.

11. The cap of any of claims 1 to 10,
wherein the distal portion has a first state and a second state,
wherein in the first state, the first arm is in a first orientation wherein the first distal end of the first arm is a first distance from a longitudinal axis defined by the tubular body, and
wherein in the second state, the first arm comprises a second orientation wherein the first distal end of the first arm is a second distance from the longitudinal axis defined by the tubular body, the second distance being greater than the first distance.

12. The cap of claim 11, wherein in the second state, the distal portion defines a second tubular body (119) adjacent to the first tubular body, wherein the lumen extends through the first and second tubular bodies.

## Patentansprüche

1. Eine Kappe (110) zur Befestigung an einem distalen Ende eines Endoskops, wobei die Kappe Folgendes umfasst:
einen proximalen Abschnitt (122), der einen röhrenförmigen Körper (114) mit einem sich dadurch erstreckenden Lumen (116) umfasst, wobei der proximale Abschnitt für die Befestigung des distalen Endes des Endoskops ausgelegt ist;
einen distalen Abschnitt (118), der einen ersten Arm (120) aufweist, der sich zu einem ersten distalen Ende erstreckt, wobei der erste Arm so beweglich ist, dass ein erstes distales Ende des ersten Arms in Bezug zu einer durch den röhrenförmigen Körper definierten Längsachse lateral beweglich ist; und
einen Auslöser (132), der den ersten Arm berührt, wobei der Auslöser beweglich ist, um die Bewegung des ersten distalen Endes des ersten Arms auszulösen;
**dadurch gekennzeichnet, dass**:
ein Kontaktabschnitt (139) des Auslösers dazu ausgelegt ist, eine erste Fläche des ersten Arms verschiebbar zu berühren; und
die Kappe ferner eine Feder (142) umfasst, die den distalen Abschnitt der Kappe umgibt und dazu ausgelegt ist, eine nach innen gerichtete laterale Kraft auf den ersten Arm in Richtung der durch den röhrenförmigen Körper definierten Längsachse bereitzustellen.

2. Kappe nach Anspruch 1, wobei der distale Abschnitt der Kappe ferner einen zweiten Arm (122) umfasst, wobei der zweite Arm als Reaktion auf die Bewegung des Auslösers beweglich ist, sodass ein zweites distales Ende des zweiten Arms in Bezug zu der durch den röhrenförmigen Körper definierten Längsachse lateral beweglich ist.

3. Kappe nach Anspruch 2, wobei der Auslöser zumindest teilweise zwischen dem ersten Arm und dem zweiten Arm angeordnet ist, wobei der Kontaktabschnitt des Auslösers in die proximale Richtung beweglich ist, um das erste distale Ende des ersten Arms von dem zweiten distalen Ende des zweiten Arms lateral abzuspreizen.

4. Kappe nach einem der Ansprüche 1 bis 3, wobei der Auslöser mit einem Kabel (146) verbunden ist, wobei das Kabel dazu ausgelegt ist, eine Kraft auf den Auslöser in einer proximalen Richtung bereitzustellen.

5. Kappe nach Anspruch 4, wobei sich das Kabel zu einem proximalen Ende eines Endoskops erstreckt.

6. Kappe nach einem der Ansprüche 1 bis 5, wobei der Auslöser eine Keilform einschließt.

7. Kappe nach einem der Ansprüche 1 bis 6, wobei der Auslöser (132) an den zweiten Arm gekoppelt ist und wobei der Auslöser drehbar ist.

8. Kappe nach Anspruch 7, wobei der Kontaktabschnitt (139) des Auslösers (132) eine Kontaktfläche einschließt, die dazu ausgelegt ist, den ersten Arm zu berühren und dem ersten Arm eine Kraft bereitzustellen, um den ersten Arm lateral von dem zweiten Arm zu trennen.

9. Kappe nach einem der Ansprüche 1 bis 8, wobei der erste Arm ein Scharnier (124) umfasst, wobei das Scharnier an dem proximalen Abschnitt der Kappe befestigt ist und eine laterale Bewegung des ersten distalen Endes des ersten Arms ermöglicht.

10. Kappe nach Anspruch 1, wobei die Feder in eine Nut (150) aufgenommen wird, die den distalen Abschnitt der Kappe umgibt.

11. Kappe nach einem der Ansprüche 1 bis 10,
wobei der distale Abschnitt einen ersten Zustand und einen zweiten Zustand aufweist,
wobei der erste Arm im ersten Zustand in einer ersten Ausrichtung ist, in der das erste distale Ende des ersten Arms einen ersten Abstand zu der durch den röhrenförmigen Körper definierten Längsachse aufweist, und
wobei der erste Arm im zweiten Zustand eine zweite Ausrichtung umfasst, wobei das erste distale Ende des ersten Arms einen zweiten Abstand zu der durch den röhrenförmigen Körper definierten Längsachse aufweist, wobei der zweite Abstand größer als der erste Abstand ist.

12. Kappe nach Anspruch 11, wobei der distale Abschnitt im zweiten Zustand einen zweiten röhrenförmigen Körper (119) definiert, der an den ersten röhrenförmigen Körper angrenzt, wobei sich das Lumen durch den ersten und zweiten röhrenförmigen Körper erstreckt.

## Revendications

1. Capuchon (110) à fixer à l'extrémité distale d'un endoscope, le capuchon comprenant :
une partie proximale (122) comprenant un corps tubulaire (114) que traverse une lumière (116),
dans lequel la partie proximale est configurée pour être solidement fixée à l'extrémité distale de l'endoscope ;
une partie distale (118) comportant un premier bras (120) s'étendant jusqu'à une première extrémité distale, le premier bras étant mobile de telle sorte qu'une première extrémité distale du premier bras peut être déplacée latéralement par rapport à un axe longitudinal défini par le corps tubulaire ; et
un actionneur (132) en contact avec le premier bras,
dans lequel l'actionneur est mobile pour mener à bien le mouvement de la première extrémité distale du premier bras,
**caractérisé en ce que** :
une partie de contact (139) de l'actionneur est configurée pour être en contact glissant avec une première surface du premier bras ; et
le capuchon comprend en outre un ressort (142) faisant le tour de la partie distale du capuchon et configuré pour exercer sur le premier bras une force latérale vers l'intérieur, vers l'axe longitudinal défini par le corps tubulaire.

2. Capuchon selon la revendication 1, dans lequel la partie distale du capuchon comprend en outre un second bras (122), le second bras étant mobile en réaction au mouvement de l'actionneur de telle sorte que la seconde extrémité distale du second bras peut être déplacée latéralement par rapport à l'axe longitudinal défini par le corps tubulaire.

3. Capuchon selon la revendication 2, dans lequel l'actionneur est disposé au moins partiellement entre le premier bras et le second bras, dans lequel la partie de contact de l'actionneur peut se déplacer dans la direction proximale pour écarter latéralement la première extrémité distale du premier bras de la seconde extrémité distale du second bras.

4. Capuchon selon l'une quelconque des revendications 1 à 3, dans lequel l'actionneur est raccordé à un câble (146), le câble étant configuré pour exercer une force sur l'actionneur dans une direction proximale.

5. Capuchon selon la revendication 4, dans lequel le câble s'étend jusqu'à l'extrémité proximale d'un endoscope.

6. Capuchon selon l'une quelconque des revendications 1 à 5, dans lequel l'actionneur présente la forme d'un coin.

7. Capuchon selon l'une quelconque des revendications 1 à 6, dans lequel l'actionneur (132) est accouplé au second bras et
dans lequel l'actionneur est rotatif.

8. Capuchon selon la revendication 7, dans lequel la partie de contact (139) de l'actionneur (132) comprend une surface de contact configurée pour être en contact avec le premier bras et exercer sur lui une force pour séparer latéralement le premier bras du second bras.

9. Capuchon selon l'une quelconque des revendications 1 à 8, dans lequel le premier bras comprend une charnière (124), la charnière étant solidement fixée à la partie proximale du capuchon et permettant un mouvement latéral de la première extrémité distale du premier bras.

10. Capuchon selon la revendication 1, dans lequel le ressort est installé dans une rainure (150) faisant le tour de la partie distale du capuchon.

11. Capuchon selon l'une quelconque des revendications 1 à 10,
dans lequel la partie distale présente un premier état et un second état,
dans lequel, dans le premier état, le premier bras est dans une première orientation dans laquelle la première extrémité distale du premier bras est à une première distance de l'axe longitudinal défini par le corps tubulaire et
dans lequel, dans le second état, le premier bras présente une seconde orientation dans laquelle la première extrémité distale du premier bras est à une seconde distance de l'axe longitudinal défini par le corps tubulaire, la seconde distance étant supérieure à la première distance.

12. Capuchon selon la revendication 11, dans lequel, dans le second état, la partie distale définit un second corps tubulaire (119) adjacent au premier corps tubulaire,
dans lequel la lumière traverse les premier et second corps tubulaires.
